Europäisches Patentamt

European Patent Office

Office européen des brevets

(19)

Publication number: **0 287 128**
**A1**

# EUROPEAN PATENT APPLICATION

(21) Application number: 88106153.5

(51) Int. Cl.⁴: **A61K 31/70**

(22) Date of filing: 18.04.88

(30) Priority: 16.04.87 US 42489

(43) Date of publication of application:
19.10.88 Bulletin 88/42

(84) Designated Contracting States:
BE CH DE ES FR GB IT LI NL SE

(71) Applicant: **Research Corporation
Technologies, Inc**
**6840 East Broadway Boulevard**
**Tucson Arizona 85710-2815(US)**

(72) Inventor: **Greer, Sheldon**
**8320 S.W. 86th Terrace**
**Miami Florida(US)**

(74) Representative: **Hansen, Bernd, Dr.rer.nat. et
al**
**Hoffmann, Eitle & Partner Patentanwälte**
**Arabellastrasse 4**
**D-8000 München 81(DE)**

(54) **Antitumor agent.**

(57) The present invention relates to a composition for treatment of cancer which comprises effective amounts of a halogenated cytidine, a cytidine deaminase inhibitor, and a uridine phosphorylase inhibitor.

EP 0 287 128 A1

## ANTITUMOR AGENT

The present invention relates to cancer chemotherapy. The invention more specifically relates to a novel composition for effective treatment of cancer.

Despite the tremendous efforts in scientific research which have been made to eradicate cancer, the disease has continued to plague mankind, with hundreds of thousands of deaths resulting each year. Substantial advances have been made in the field of chemotherapeutics, which has rapidly become a highly successful adjunct to radiation and surgery, and has been responsible for prolonging the lives of many cancer victims. However, the typical problem which arises with chemotherapeutic agents is that those which most effectively destroy or inactivate tumor cells often have similar effects on normal cells. This nonspecificity for tumor cells results in the often severe side effects, such as bone marrow suppression, gastrointestinal problems, including nausea, neurological disorders, and alopecia, which have come to be routinely associated with chemotherapy treatments.

The search for a suitable chemotherapeutic agent which will effectively destroy malignant cells, but which will have limited or no ill effects on normal cells has been a subject of intense studies in recent years. The focus has been on finding differences between cancerous and normal cells and exploiting those differences for a chemotherapeutic advantage.

A number of existing anticancer agents operate by interfering with the biosynthesis or function of nucleic acids, essential components of the cells' DNA and RNA. Among the classes of compounds which fall into this category are alkylating agents, inhibitors of the mitotic apparatus and antimetabolites. The latter group of compounds, the antimetabolites, have received particular attention in recent years. These agents function by interfering with normal nucleic acid synthesis by competing with the normal metabolites for incorporation into nucleic acids or by acting as inhibitors of enzymes involved in nucleic acid biosynthesis. Since successful cell division depends upon normal DNA replication, transcription and translation, this attack at the nucleic acids predictably has an effect on the growth of tumor cells. The compounds which are contemplated as being useful as antimetabolites are typically purine and pyrimidine base analogues, or their nucleosides, and it is in this area that much of the current research has been concentrated.

For example, U.S. Patent Nos. 4,472,386 and 4,490,366 both relate to fluorouridine (FUrd) derivatives claimed to be useful in tumor therapy. The compound 5-fluorouracil (FUra) has also long been used as a chemotherapeutic agent (Heidelberger et al., Cancer Research 18:305, 1958), and others halogenated uracil derivatives have also been described (U.S. Patent Nos. 4,415,573; 4,371,535 and 4,340,728). Similarly, the compound 5-fluorodeoxycytidine (FdC) has been suggested as having potential for antineoplastic properties (Newman, et al., PNAS USA 79: 6419-6423, 1982), and Mekras and Greer, Fed. Proc. 42: 360, 1983) have described its neoplastic properties. Although a number of these compounds such as 5-fluorouracil or 5-fluorodeoxyuridine are indeed rather effective in killing tumor cells, they unfortunately do not show any substantial selectivity between normal cells and tumor cells. Thus, the regression of a tumor is rarely unaccompanied by relatively severe side effects due to the toxicity to normal tissues. Furthermore, many of these compounds, especially the deoxyuridine or uridine analogs, may be degraded by enzymes in the serum and in the liver so that there is less effective tumor inhibition. FdC coadministered with $H_4U$ has shown somewhat improved selectivity; however, the results are not comparable to that obtained with the combination reported in the present invention.

In order to avoid the problems resulting from lack of selectivity, a number of compositions have been prepared which comprise the active antimetabolite and an additional compound which theoretically aids in favoring the activity of the compound in the tumor cells alone; this scheme is generally known as "metabolic channelling". For example, benzacyclouridine (BAU) and derivatives have been used in conjunction with FdUrd (Chu, et al., Cancer Res. 44:1852-56, 1984; U.S. Patent No. 4,613,604). The presence of the BAU is said to improve the activity of the FdUrd by inhibiting one of two possible enzymes, which routinely convert it to the less toxic FUra; since one of the two enzymes responsible for this action is present in only very low levels in tumor cells relative to normal cells, the pathway is completely inhibited, whereas in normal cells the second enzyme continues to degrade the FdUrd, thereby reducing its toxicity to normal cells. Similarly, tetrahydrouridine ($H_4U$) has been employed in conjunction with 5-trifluoromethyl deoxycytidine ($F_3$methyldC; Mekras et al., Cancer Res. 45:5270-80, 1985); 5-chlorodeoxycytidine (CldC) (Perez et al., Int. J. Rad. Oncol. Biol. Phys. 10:1453-1458, 1984; and FdC (Mekras et al., Cancer Res. 44:2551-2560, 1984). The rationale for this combination is to exploit higher levels of the enzymes cytidine deaminase and deoxycytidylate deaminase in tumor cells, so that the $H_4U$ effectively inhibits cytidine deaminase in normal cells but only moderately inhibits the enzyme in tumor cells; the halogenated pyrimidine analogue is then preferentially incorporated into the DNA and RNA of tumor cells. The aforementioned combination of agents does to some extent circumvent the problems of selectively affecting only tumor cells. However, even though these combinations may aid in more effectively distributing the toxic effects of the active compounds to tumor cells, there are still certain undesirable residual effects on normal cell, for example, uridine depletion. Thus, side effects in normal cells are still not satisfactorily eliminated by these combinations, and a need continues to exist for a chemotherapeutic agent which can effectively destroy tumor cells while substantially avoiding the effects of toxic antimetabolites in normal tissue.

In this regard, the present invention provides a novel pharmaceutical composition which fulfills the stated requirements of a desirable chemotherapeutic agent. The present composition, which is a combination of three different compounds, exhibits an extraordinary effectiveness in the destruction of tumor cells and regression of tumors, at a level equal to or greater than any of the aforementioned agents; at the same time, the composition acts in such a manner as to substantially decrease the harmful effects of the active ingredient on normal cells, so that non-tumor associated side effects are minimized to a greater extent than has previously been possible with known chemotherapeutic agents.

Figure 1 shows the effectiveness of the present compositions on treatment of Lewis Lung carcinoma, in comparison with other known anticancer compositions.

1A represents the control;

1B represents treatment with FUra;

1C represents treatment with FUra + BAU;

1D represents treatment with FdC and H₄U;

1E represents treatment with the present composition.

Each line represents an individual mouse.

Figure 2 compares the effectiveness of the present compositions in treatment of Lewis Lung carcinoma, with that of FdC + H₄U, FdU + H₄U. and FdU + H₄U + BAU.

The present invention relates a pharmaceutical composition in dosage unit form comprising effective amounts of a hologenated cytidine or deoxycytidine, a cytadine deaminase inhibitor, and a uridine phosphorylase inhibitor.

The present invention also relates to a method of inducing tumor regression in a vertebrate which comprises administering to the vertebrate effective amounts of a halogenated cytidine, a cytidine deaminase inhibitor, and a uridine phosphorylase inhibitor.

The present composition employs three distinct compounds, a halogenated cytidine (HalC) or deoxycytidine (HaldC), a cytidine deaminase inhibitor and a uridine phosphorylase inhibitor, in combination, to achieve a superior therapeutic affect on tumor cells. The cytidine compound is the active pyrimidine analogue which is ultimately responsible for toxicity in tumor cells. However, in order to achieve the desired effect of destroying tumor cells while leaving normal cells substantially unharmed, the other two components, which themselves have no anti-tumor action, must be present. As was shown in the foregoing discussion, none of the components of the composition are novel, and each has been used previously in some form of anticancer composition, whether alone, or in combination with other agents. For example, as noted above, FdC has been known to be used with H₄U, a cytidine deaminase inhibitor by the inventor; and BAU, a uridine phosphorylase inhibitor, has been used in conjunction with FUra, all for cancer treatment. However, it has now been surprisingly discovered that the specific combination of halogenated cytidine or deoxycytidine, a cytidine deaminase inhibitor and a uridine phosphorylase inhibitor, has an effect which is far superior to any of these previously disclosed combinations, particularly in promoting a remarkable extent of tumor cell kill which results in long term cures of the animal, and secondarily with respect to reducing toxic effects on normal cells. These unexpected increases in therapeutic properties appear to be the result of a precise and favorable interaction between those specific elements, which could not be predicted from the previously known effect of the compounds alone or in combination with other compounds.

The present composition exerts its ultimate effect by preferential tumor directed conversion of a nontoxic nucleoside analog to a toxic antimetabolite by enzymes which are markedly elevated in tumor tissue. In the present case, the elevated enzymes involved in the pathway of interest are cytidine deaminase, deoxycytidine kinase, and deoxycytidylate deaminase; the latter enzyme is particularly elevated, from 20-80 fold, in most human tumors including colorectal, lung, breast, brain and melanoma. DNA and RNA-directed antimetabolites are produced via the cytidine deaminase pathway which converts the nontoxic fluorocytidine or fluorodeoxycytidine to the toxic compound FUMP and FdUMP by conversion of FdU via thymidine kinase, thus inhibiting normal maturation of the RNA and resulting in inhibition of DNA synthesis and extensive repair of DNA. DNA-directed antimetabolites are also produced via the cytidine kinase and cytidylate deaminase pathway, which produces the toxic FdUMP from nontoxic fluorodeoxycytidine. To a certain extent this aspect of the metabolic events involved in therapy does not differ significantly from that observed with the use of other nucleoside analogue compositions, in that the cells' normal metabolic pathways are exploited to convert nontoxic analogues to toxic analogues which inhibit DNA synthesis for incorporation into the tumor cells' nucleic acids. However, a significant advantage of the present composition is that the undesirable effect of normal cell starvation is substantially avoided.

Uridine starvation in normal cells is due, in part, to the complete inhibition of cytidine deaminase which takes place most extensively in those cells. Cytidine deaminase which converts cytidine to uridine is an important source of uridine. The issue of uridine starvation is of even greater importance in view of the fact that the normal cells contain the uridine analog, FUMP. This incorporation of FUMP into RNA has deleterious effects on the cell. This incorporation can be antagonized by uridine and is more extensive in the absence of uridine. Furthermore, uridine serves as an ultimate precursor for thymidine, which antagonizes the DNA-directed effects of the fluorinated pyrimidine analogs. The three components of this invention are uniquely required because of the novelty of their individual and collective mode of action.

Thus, the inhibitor of uridine phosphorylase extends the half-life of $H_4U$, favors its distribution to normal cells and prevents the degradation of uridine which is so important to these cells which face problems of uridine starvation in view of the inhibition of an important source of uridine (cytidine deaminase); further, superimposed on all of these factors is the presence of fluorinated uridine analogs in this cells.

The combination of a uridine phosphorylase inhibitor with the other two components has the previously unknown benefit of preventing uridine depletion in normal cells, while also providing the unexpected desirable result of favoring a preferential distribution of the cytidine deaminase inhibitor to normal tissue and extend its half life. This pairing of effects permits the use of higher levels of the cytidine deaminase inhibitor to act more efficiently in protecting normal cells and providing tumor directed toxicity. The ultimate result of this unique combination of ingredients is most clearly reflected when the relative number of cures, and reduced weight loss, in animals treated with the present composition are compared with animals treated with known combinations.

While not wishing to be bound by any particular mechanism of action, it appears that one advantage of the present invention is that the inhibitor of uridine phosphorylase not only affects the metabolism of the fluorinated pyrimidines towards tumor selectivity, but superimposed on this is an effect on the modulator (tetrahydrouridine), which inhibits cytidine deaminase, since it too is a substrate of uridine phosphorylase.

This synergistic interaction results in a surprising and extraordinary superiority over the fluorinated pyrimidines in current use in cancer chemotherapy (FUra or FdU) even when they are coadministered with an inhibitor of uridine phosphorylase. 50 to 80%, usually 70% cures are obtained with the combination under conditions where no cures are obtained with the fluorinated pyrimidines in current use. This unusual frequency of cures occurs with less than 10% weight loss.

Another unusual advantage of the invention is that the problem of tumors becoming refractory to drug therapy is minimized by the fact that FdC, for example, is metabolized by two separate alternative pathways. Should mutation towards drug resistance develop because of the loss of one enzyme, then the cells carrying the mutation would still be sensitive to therapy and would not become the dominant population.

Another advantage of the invention is that one of the metabolic products of FdC, FdCTP, when incorporated into DNA, will result in the cessation of growth of those cells by a completely different mechanism than that in operation with the 5-fluorinated pyrimidine analogs in current use.

The incorporation of FdC (and F3methyldC) into tumor DNA (which is highly selective) results in a change in the methylation status of the DNA of those cells which results in the expression of differentiated functions which is mutually exclusive to rapid cell division. This mechanism is superimposed on the three mechanisms of inhibition of tumor cell growth which are also in force with the fluorinated pyrimidine in current use: a) inhibition of the maturation of ribosomal RNA, b) inhibition of a key enzyme in the synthesis of DNA precursors (thymidylate synthetase), c) incorporation of 5-fluorouracil into DNA and its subsequent removal from DNA by repair enzymes which results in the fragmentation of DNA.

The present combination requires the use of a halogenated cytidine or deoxycytidine as the pyrimidine analogue. When used generically in the present specification and claims, the term "cytidine" is intended to encompass both cytidine per se and deoxycytidine. The substitution of FUra, FdUrd, FUrd or $F_3dT$ will not provide the same superior results. The halogen is selected from bromine, chlorine, iodine and fluorine, with fluorine being the preferred halogen. The halo atoms may also form part of another substituent on the cytidine molecule, for example, a halogenated 5-alkyl such as 5-trifluoromethyl. It is preferred that the halo be present in at least the 5-position of the cytidine molecule. Either cytidine and deoxycytidine may be used as the base compound. The preferred active ingredients are 5-fluorodeoxycytidine, trifluoromethyldeoxycytidine, 5-chlorodeoxycytidine and 5-fluorocytidine, with 5-fluorodeoxycytidine and 5-trifluoromethyldeoxycytidine particularly preferred.

4

The second component of the present composition is a cytidine deaminase inhibitor. Although any of the known inhibitors may be acceptable, it is particularly preferred to employ tetrahydrouridine or, less preferably, deoxytetrahydrouridine for this purpose. Other compounds which may alternately be employed are pyrimidin-2-one nucleosides and, less preferably, deazauridine.

The uridine phosphorylase inhibitors useful in the present composition are preferably selected from the known acyclonucleosides. The most preferred inhibitor is benzacyclouridine, or any of the known derivatives of benzacyclouridine, such as benzyloxy-benzylacyclouridine, or hydroxymethyl derivatives of either of these compounds, as disclosed in U.S. Patent No. 4,613,604. However, other useful uridine phosphorylase inhibitors include deoxyglucosyl thymidine, or 5-substituted 2,2'anhydrouridines such as 5-ethyl 2,2' anhydrouridine and less preferably 1-m-methoxybenzyluracil, 5-benzyluracil, 1-deazauracil, acyclothymidine and 6-amino-5-halouracils, such as 5-bromo-6-amino-uracil (uridine). Other potentially useful inhibitors are 2,6-pyridinethiol; 6-benzo-2-thiouracil, 5-nitrouracil (uridine); 1-($\beta$-L-p-2-deoxyribopyranosyl) thymine; 1-(2-deoxy-$\beta$-D-glucopyranosyl) thymine; 5-benzyl-4,5-benzyloxy-2'-deoxyglucosyl uracil; and 5-benzyl-1-deazauracil.

The amount of each component used in a single dose of the present composition may vary in accordance with which specific cytidine component is used. Because FdC is the most potent of the proposed compounds, relatively small doses are required to achieve the desired effect. Generally a range of about 8-15 mg/kg of body weight of the treated individual is sufficient, with 10-12 mg/kg being the preferred range. Similar doses are employed with 5-fluorocytidine: the dose of 5-fluorocytidine would be utilized in a range of 1 to 10 mg/kg with 2 to 5 mg/kg being the most preferred dosage. When used in conjunction with an inhibitor like FdC, the cytidine deaminase inhibitor, such as $H_4U$, should be present in the amount of about 10-50 mg/kg, with the preferred range being about 15-30 mg/kg and the most preferred dose being about 20-25 mg/kg. On the other hand, when a less potent cytidine component, such as $F_3$methyldC is used, the dosage of each of these elements must be increased. For example, the preferred range of $F_3$methyldC is about 10-300 mg/kg, with 75-225 being a preferred range, and 175-210 mg/kg being the most preferred dosage. $H_4U$ must also be increased, to approximately twice the amount of $H_4U$ employed with FdC. It is within the skill of the experienced practitioner to vary the dosages of each of these components in accordance with the potency of the cytidine component employed. In any case, the amount of uridine phosphorylase inhibitor, such as BAU, used may remain relatively constant, in the range of about 5-50 mg/kg, with 8-35 being a preferred range, and 10-30 being most preferred.

The present composition may be administered in any manner which is traditional for administration of chemotherapeutic agents, e.g., as capsules or tablets for oral administration. When oral preparations are desired, the compounds may be combined with the typical carriers, such as lactose, sucrose, starch, talc magnesium stearate, crystalline cellulose, methyl cellulose, hydroxypropyl cellulose, hydroxypropylmethyl cellulose, carboxymethyl cellulose, glycerin, sodium alginate, or gum arabic among others. For parenteral administration, suitable carriers are water or physiological saline.

The periodicity of the administration of the composition may be varied in accordance with the specific type and severity of the condition to be treated, and these variations will be apparent to the treating physician. The individual components of the effective composition may in fact be administered separately, but it is preferable to administer these as a unit dosage composition. A typical pattern may include daily administration of the prescribed dosage for a period of about a week, with the possibility, after a rest period of one week, of a repeat of the cycle for an additional week. The treatment may also be used as an adjunct to a treatment regimen including surgery and/or radiation treatments.

The present composition may be used in the treatment of any type of tumors for which 5-FUra or FUrd treatment has traditionally been recommended. Among these are colorectal and breast cancer. The strategy of the present invention would be most effective against many of the commonly occuring human cancers because of the elevation of cytidine deaminase and/or dCMP deaminase in those human tumors. These include: adenocaricnoma of the breast, bronchiogenic carcinoma of the lung, adenocarcinoma of the colon and rectum astrocytama of the brain melignant melanoma carcinoma because of its apparently distinct method of operation, the present composition and method may also be effectively employed with tumors typically resistant to FUra and FUrd treatment. As used in the present specification and claims, "tumor regression" is intended to be understood as affecting all stages of tumor development, i.e., preventing further tumor growth, as well as actively destroying the tumor cells. The present compositions are also intended for use as radiosensitization of tumor cells, in a manner similar to that described by Perez et al. - (Int. J. Rad. Oncol. Biol. Phys. 10:1453-1458, 1984) for CIC and $H_4U$ and the use of the compositions in conjunction with radiotherapy in this specific manner is also intended. The purpose of such treatment is to permit smaller doses of radiation to be used, thereby reducing the use of radiation damage to adjacent normal tissue. This mode of treatment may be effectively employed with either X-ray or gamma radiation.

The present invention may be better understood by reference to the following non-limiting examples.

## EXAMPLE 1 - 5

The following examples demonstrate the effective use of the present compositions.

In Examples 1 to 5, $BD_2F_1$ mice were implanted with 2.2-3 mm tumor fragments bilaterally in the auxiliary region via inguinal puncture on day 0. The tumor tissue was obtained from mice bearing 7 to 10 day old tumors. To obtain the tumors, the animals were sacrificed by cervical dislocation. Tumors were removed aseptically and placed into petri dishes containing ice-cold phosphate buffered saline. Tumors were debrided, necrotic tissue was removed and viable tissue was cut into 2-3 mm fragments. These fragments were loaded into 13-gauge trochars for implantation. Animals were weighed prior to each i.p. injection, which began one day after implantation unless otherwise noted, so that the volume of injection could be adjusted to their individual weight.

## EXAMPLE 1A

Animals were implanted with Lewis lung carcinoma. Five animals were given FdC (12mg/kg) + $H_4U$ 25mg/kg + BAU 9mg/kg 1 day $\times$ 7 days starting on day 1. Comparisons included FUra (25mg/kg) and FUra + BAU (10mg/kg).

## EXAMPLE 1B

Animals were implanted with Lewis lung carcinoma. Seven animals were given FdC (12mg/kg) + $H_4U$ (25mg/kg) + BAU (30mg/kg). Comparisons included FUra 25 mg/kg and FUra + BAU (30mg/kg). The treatment schedule was as in Example 1A.

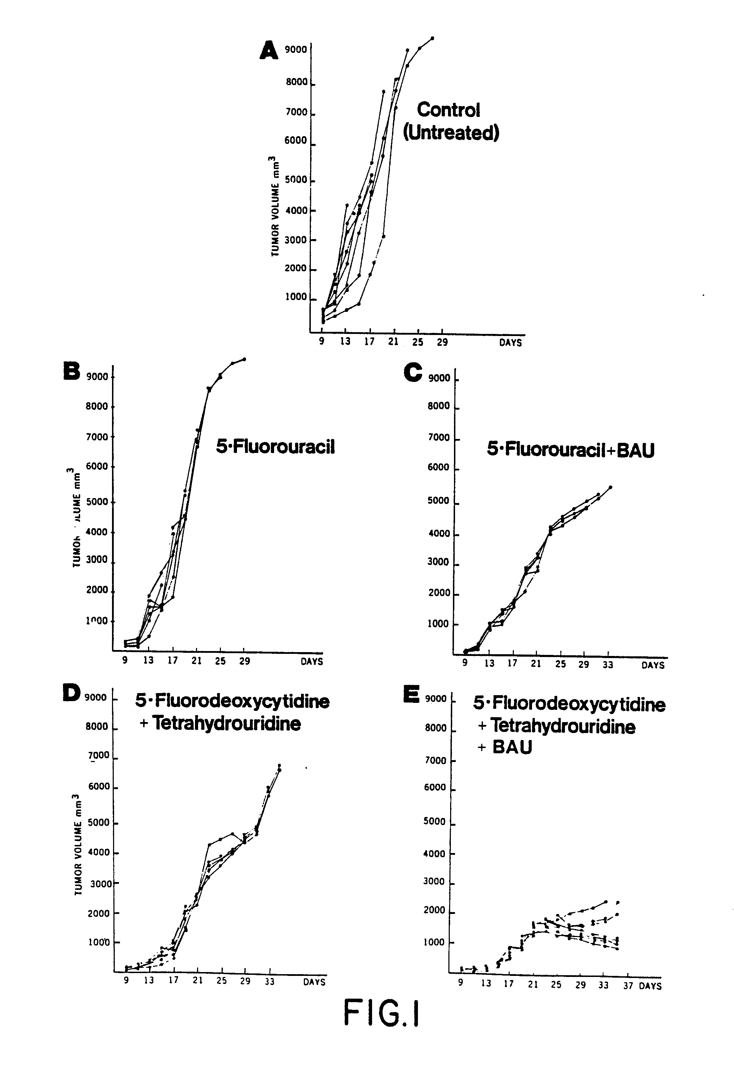

The results of experiments 1A and B are summarized in Table 1, and experiment B is also summarized in Figure 1. Seven of twelve cures were obtained with the combination of the invention. No cures were obtained with 5-fluorouracil, which was tested at its maximum tolerated dose. 5-fluorouracil, the agent in current use, was also relatively ineffective even when BAU was added. Only one of thirteen cures was obtained with FdC + $H_4U$.

TABLE 1 (see Figure 1)

Lewis Lung Carcinoma

| Condition in Accompanying Figure | Maximum Wgt. Loss % | | Mean Day of Death Not including cures | | T/C | | T/C* | | No. Cures/No. Mice | |
|---|---|---|---|---|---|---|---|---|---|---|
| | Exp. 1 | Exp. 2 | Exp. 1 | Exp. 2 | Exp. 1 | Exp. 2 | Exp. 1 | Exp.2 | Exp. 1 | Exp.2 |
| A Control | | | 17 | 21 | | | | | 0/7 | 0/8 |
| B FUra 25 mg/kg | 18 | 21 | 20 | 25 | 1.19 | 1.17 | | | 0/5 | 0/6 |
| C FUra + BAU‡ 25 mg/kg | 18 | 17 | 32 | 30 | 1.92 | 1.41 | | | 0/6 | 0/6 |
| D FdC + H4U 12 mg/kg 25 mg | 9.9 | 10 | 30 | 36 | 1.79 | 1.70 | >2.1 | | 1/6 | 0/7 |
| E FdC + H4U + BAU‡ 12 mg/kg 25 mg/kg | 7.1 | 8.1 | 41 | 45 | 2.44 | 2.10 | >3.1 | >4.2 | 3/5 | 4/7 |

*T/C assigning cures a 60 day life span.

‡Dose of BAU was 9 mg/kg in experiment 1 and 30 mg/kg in experiment 2. Treatment schedules were 1/day x7, 1 day after bilateral tumor implantation.

0 287 128

## EXAMPLE 2

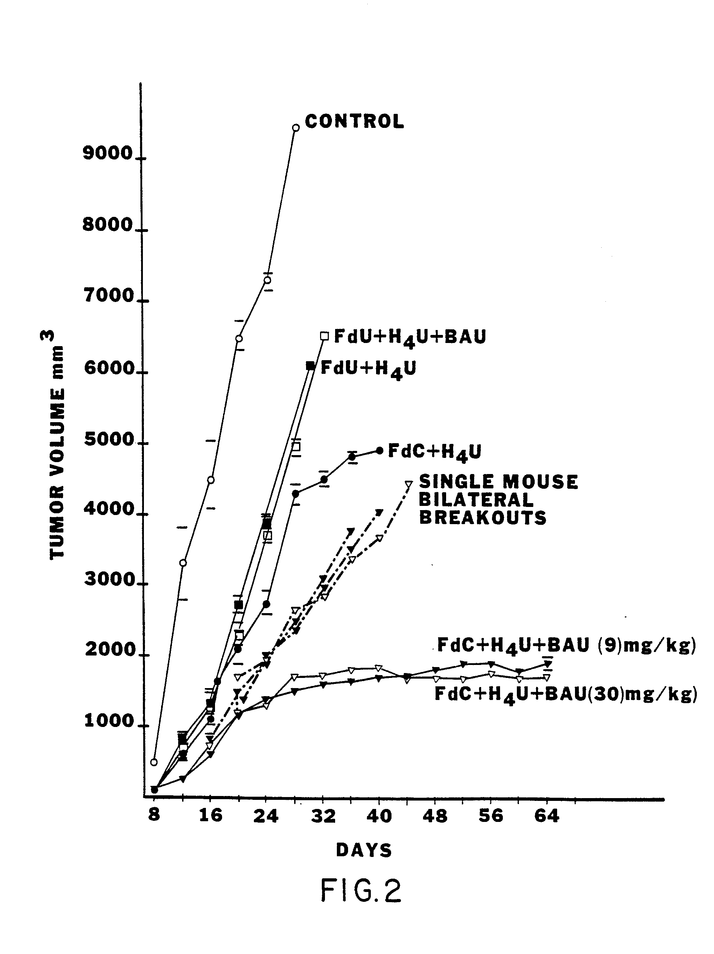

Animals were implanted with Lewis. lung carcinoma. The treatment schedule was as described above. A comparison was made with FdU tested at its maximum tolerated dose, 75mg/kg. FdU was not effective when administered with $H_4U$ (T/C of the mean day of death = 0.97) and was only slightly more effective when BAU was added (T/C = 1.1) and no cures were obtained unless FdC + $H_4U$ + BAU was administered. Nine of 12 cures were obtained with the combination of the invention. The data is summarized in Table 2 and Figure 2. "Day of breakout" indicates the day tumor growth was first noticed in a group of animals in which tumor growth was arrested or in which tumor regression occurred. Breakouts or cures are always bilateral, which indicates an involvement of a host response.

Not shown in the table are the results of a study with a group of animals which received BAU (30mg/kg), $H_4U$ (25mg/kg) alone and BAU + $H_4U$. There was no significant tumor inhibition achieved, no increase in the mean day of death, and no cures were obtained with these agents when administered without a fluorinated pyrimidine analog.

Table 2 (see Figure 2)

Lewis Lung Carcinoma:

| Condition (mg/kg) | Day 10 14 18 28 38 67 % Tumor Inhibition | | | | | | Max. % Wgt. Loss | # Toxic Deaths/6 | Mean Day of Death | T/C | # Cures /6 | Day of Break-out |
|---|---|---|---|---|---|---|---|---|---|---|---|---|
| FdC + H4U (12) (25) | 88 | 77 | 63 | 55 | | | 10 | 0 | 32.3 | 1.5 | 0 | -- |
| FdC + H4U + BAU (12) (25) (9) | 89 | 89 | 83 | 82 | 81 | 80 | 8.7 | 0 | 38.0 | 1.8 | 4 | 16, 21 |
| FdC + H4U + BAU (12) (25) (30) | 86 | 89 | 83 | 82 | 81 | 82 | 8.4 | 0 | 44 | 2.1 | 5 | 20 |
| FdU + H4U (75) (25) | 84 | 75 | 65 | 36 | | | 14 | 0 | 20.7 | 1.0 | 0 | -- |
| FdU + H4U + BAU (75) (25) (9) | 89 | 77 | 67 | 48 | | | 13 | 0 | 23.6 | 1.1 | 0 | -- |
| Control | | | | | | | | | 20.7 | | | |

## EXAMPLE 3

Mice were implanted bilaterally with Lewis lung carcinoma (see Table 3). The dose of FdU was lowered to 70mg/kg, and again, it was not as effective when coadministered with H₄U + BAU in contrast to the effectiveness of FdC + H₄U + BAU. The dose of FdU was lowered from that utilized in Example 2 in response to the finding that elevation of the dose of FdC to 14mg/kg resulted in diminished tumor inhibition, mean day of death and the absence of cures compared to a dose at 12mg/kg in a protocol in which $H_4U$ and BAU was coadministered.

I = one cycle

II = two cycles

In Example 3 it can be seen that the administration of FdC + $H_4U$ for 1 day for 7 days beginning on day 1 followed by 6 days of no treatment followed, in turn, by 7 days of treatment resulted in an increase in the mean day of death T/C from 1.70 for one cycle to 2.03 for two cycles; however, no cures were obtained. In contrast, the coadministration of BAU (30mg/kg) in a protocol utilizing one cycle of treatment resulted in a T/C of 2.05 (not including cures) and 3/6 cures. A two cycle schedule with FdC + $H_4U$ + BAU resulted in a T/C of 2.77 and 4/6 cures. A repeat of the two cycle experiment (7 on, 6 off, 7 on) utilizing FdC (12mg/kg), $H_4U$ (25mg/kg) and BAU (10mg/kg) resulted in 14/20 or 70% cures with only 9% weight loss. In the same experiment, a one cycle treatment also resulted in 14/20 cures with only 9% weight loss.

## TABLE 3

### Lewis Lung Carcinoma

| Condition (mg/kg) | Day 10 | 14 | 18 | 22 | 26 | 38 | 42 | Maximum % Weight Loss | # Toxic Deaths /6 | Mean Day of Death | T/C | # Cures /6 | Day of Break-out |
|---|---|---|---|---|---|---|---|---|---|---|---|---|---|
| | % Tumor Inhibition | | | | | | | | | | | | |
| FdU (70) | 83 | 74 | 58 | 42 | 31 | 19 | | 19 | 0 | 26.0 | 1.24 | 0 | |
| FdU + H4U + BAU (70) (25) (30) | 90 | 82 | 80 | 74 | 68 | 62 | 56 | 12 | 0 | 38.1 | 1.81 | 0 | |
| I FdC + H4U (12) (25) | 83 | 85 | 72 | 65 | 62 | 57 | 40 | 10 | 0 | 36.0 | 1.70 | 0 | |
| II FdC + H4U (12) (25) | 83 | 85 | 75 | 70 | 66 | 61 | 52 | 11 | 0 | 42.9 | 2.03 | 0 | |
| I FdC + H4U + (12) (25) BAU (30) | 88 | 90 | 85 | 81 | 81 | 80 | 81 | 8.8 | 0 | 43.3* | 2.05* | 3 | 15, 22, 38 |
| II FdC + H4U + (12) (25) BAU (30) | 89 | 90 | 88 | 86 | 84 | 84 | 83 | 9.0 | 0 | 68.5* | 2.77* | 4 | 34, 54 |
| Control | | | | | | | | | | 21.1 | | | |

*not including cures

### EXAMPLE 4

Animals were implanted with Lewis lung carcinoma (see Table 4). BAU markedly enhanced the efficacy of 5-trifluoromethyl deoxycytidine (F₃methyldC) coadministered with H₄U. 3/6 cures were obtained with the combination of agents. In addition, extensive and sustained tumor inhibition was achieved, and there was a substantial increase in the mean day of death (T C = 1.93).

In a repeat of this experiment with F₃methyldC and H₄U utilizing 30mg rather than 10mg of BAU/kg and two cycles of treatment (7 on, 6 off, 7 on) 6/10 cures were obtained with only 9.4% weight loss.

The efficacy of 5-fluorocytidine (the ribonucleoside) coadministered with H₄U is also enhanced by the addition of BAU, although no cures were obtained.

## TABLE 4
### Lewis Lung Carcinoma

| Condition | mg/kg | Day: 8 | 12 | 16 | 20 | 24 | 28 | 30 | )) | 62 | % weight loss | # toxic deaths/6 | day of death | mean day of death | T/C | # of cures/6 |
|---|---|---|---|---|---|---|---|---|---|---|---|---|---|---|---|---|
| F₃CH₃dC<br>H₄U<br>BAU | 200<br>50<br>30 | 95 | 95 | 91 | 91 | 88 | 86 | 86 | 86 | 62 | 9.1 | 0 | 30+,36+,59+++ | 41.6** | 1.93 | 3 |
| F₃CH₃dC<br>H₄U | 200<br>50 | 95 | 95 | 90 | 83 | 77 | 70 | 66 | -- |  | 8.9 | 0 | 18,24,28<br>30,42,46 | 31.3 | 1.45 | 0 |
| FrC<br>H₄U<br>BAU | 2<br>25<br>10 | 80 | 76 | 65 | 61 | 51 | 41 | 36 | -- |  | 12.1 | 0 | 20,24,26<br>30,42,46 | 31.3 | 1.45 | 0 |
| FrC<br>H₄U | 2<br>25 | 78 | 70 | 60 | 51 | 43 | 33 | 29 | -- |  | 12.8 | 0 | 16,20,26<br>28,30,46 | 27.7 | 1.20 | 0 |
| Control |  |  |  |  |  |  |  |  |  |  |  |  | 13,16,18,19<br>23,25,29,30 | 21.6 |  |  |

\* Day of breakout = 20
++ Day of breakout = 28
+++ Day of breakout = 50
** not including cures

12

EXAMPLE 5

Mammary adenocarcinoma-755 was implanted bilaterally as a solid tumor in BD2F· mice (see Table 5). The administration of drugs 1/day for 7 days followed by five days of rest, followed in turn by seven days of treatment, resulted in 3/6 cures against this tumor which is usually only poorly responsive to fluorinated pyrimidines. The one death which occurred on day 12 may be attributed to an unusually unresponsive animal or to an injection trauma death rather than to toxicity.

TABLE 5

Mammary Adenocarcinoma-755 (solid)

| Condition (mg/kg) | % weight loss | # of toxic deaths/6 | day of death | mean day of death | T/C | # of cures/6 |
|---|---|---|---|---|---|---|
| FdC + H₄U (12) (25) one cycle | 11 | 0 | | 24.7 | 1.4 | 0 |
| FdC + H₄U + BAU (12) (25) (10) one cycle | 11 | 0 | | 28.8 | 1.7 | 0 |
| FdC + H₄U (12) (25) two cycles | 12 | 0 | | 32.0 | 1.8 | 0 |
| FdC + H₄U + BAU two cycles | | | 12,30,34 | | | 3 |
| FdU + BAU (70) (10) | 10 | 0 | | 25.5 | 1.5 | 0 |
| FUra + BAU (25) (10) | 9 | 0 | | 27.5 | 1.6 | 0 |
| Control | | | | 17.1 | | |

EXAMPLE 6

An inoculum of $10^7$ cells of mammary adenocarcinoma-755 in its ascitic form was injected i.p. into BD2F· mice. Forty-eight hours later (day 2) treatment 1/day × 7) was initiated. The results in Table 6 show that, with this extreme challenge, 3/6 cures were obtained with FdC (12mg/kg) + H₄U (25mg/kg) and BAU (10mg/kg). No cures were obtained with FUra ± BAU and with FdC + H₄U.

When lower inocula were utilized ($10^5$ or $5 × 10^5$ cells), the combination of the invention was again shown to be far superior to FUra ± BAU and superior to FdC + H₄U as seen Table 7. A striking superiority of the present composition to FUra is especially apparent upon examining the mean day of death.

## TABLE 6
### Mammary Adenocarcinoma - 755, Ascitic

| Condition | % weight loss | % toxic deaths/6 | day of death | mean day of death | T/C | # of cures/6 | Approximate day of breakout |
|---|---|---|---|---|---|---|---|
| FdC (12) H$_4$U (25) BAU (10) | 9.4 | 0 | 37,42,68 | | | 3 | 31,34,50 |
| FdC (12) H$_4$U (25) | 10.2 | 0 | 29,31,36 42,59,62 | 43.1 | 3.08 | 0 | |
| FUra (25) BAU (10) | 14.4 | 0 | 12,14,17 32,42,46 | 27.1 | 1.94 | 0 | |
| FdU (60) BAU (10) | 15.2 | 0 | 11,14,16 32,35,42 | 25.0 | 1.79 | 0 | |
| Control | | | 9,11,14,14 15,17,19 | 14.0 | | | |

## Table 7

### Ascitic Mammary Adenocarcinoma-755

| Condition (mg/kg) | Size of Inoculum | Maximum Weight Loss* | Day of Death | Mean day of Death | T/C** | # Cures /6 mice | % Cures |
|---|---|---|---|---|---|---|---|
| FdC H$_4$U (12) (25) | $10^5$ | 13.7 | 33,36 | 34.5 | 2.72 | 4 | 67 |
| | $5 \times 10^5$ | 13.9 | 32,35,37 | 34.7 | 2.73 | 3 | 50 |
| FdC H$_4$U BAU (12) (25) (10) | $10^5$ | 13.5 | 34 | 34 | 2.67 | 5 | 83 |
| | $5 \times 10^5$ | 13.7 | 34,37 | 35.5 | 2.80 | 4 | 67 |
| FUra | $10^5$ | 17.0 | 17,19 21,33 | 20.0 | 1.57 | 2 | 33 |
| | $5 \times 10^5$ | 17.4 | 17,18 21,22 | 19.2 | 1.51 | 1 | 17 |
| FUra BAU (26) (10) | $10^5$ | 17.1 | 17,19,23 | 19.7 | 1.55 | 3 | 50 |
| | $5 \times 10^5$ | 17.3 | 16,17 19,21 | 18.3 | 1.44 | 2 | 33 |
| Control | $5 \times 10^5$ | | 9,9,12,12 13,14,15,16 | 12.5 | | | |

\* No toxic deaths occurred
** Not including cures

## Claims

1. A pharmaceutical composition in dosage unit form, said composition comprising effective amounts of a halogenated cytidine or deoxycytidine, a cytidine deaminas inhibitor, and a uridine inhibitor.

2. The composition of Claim 1 wherein the cytidine is 5-fluorodeoxycytine or 5-trifluoromethyldeoxycytidine or 5-fluorocytidine.

3. The composition of Claim 1 or 2 wherein the cytidine deaminase inhibitor is tetrahydrouridine and the uridine phospharylase inhibitor is 5-benzylacyclouridine.

4. The composition of Claims 2 and 3 wherein the composition contains 5-fluorodeoxycytidine, tetrahydrouridine and 5-benzylacyclouridine in such concentration ratios as to provide dosage units containing from about 8-15 mg/kg of body weight of 5-fluorodeoxycytidine; from about 10-50 mg/kg of body weight of tetrahydrouridine and from about 5-50 mg/kg of body weight of 5-benzylacyclouridine.

5. The composition of Claims 2 and 3 wherein the composition contains 5-trifluoromethyldeoxycytidine, tetrahydrouridine and 5-benzylacyclouridine in such concentration ratios as to provide dosage units containing from about 10-300 mg/kg of body weight of 5-trifluoromethyldeoxycytidine, from about 20-100 mg/kg of body weight of tetrahydrouridine and from about 5-50 mg/kg of body weight of 5-benzylcyclouridine.

6. The composition of any of Claim 1 to 5 wherein the composition contains a pharmaceutically acceptable carrier.

7. A kit comprising a carrier being compartmented to receive a series of containers in close confinement which comprises:

    (a) a first container containing 5-fluorodeoxycytidine;

    (b) a second container containing tetrahydrouridine; and

    (c) a third container containing 5-benzylacycylouridine;

    said compounds being present in such concentration ratios as to provide dosage units containing from about 8-15 mg/kg of body weight of 5-fluorodeoxycytidine, from about 10-50 mg/kg of body weight of tetrahydrouridine and from about 5-50 mg/kg of body weight of 5-benzylacyclouridine.

8. A kit comprising a carrier being compartmented to receive a series of containers in close confinement which comprises:

    (a) a first container containing 5-trifluoromethyldeoxycytidine;

    (b) a second container containing tetrahydrouridine; and

    (c) a third container containing 5-benzylacylouridine;

    said compounds being present in such concentration ratios as to provide dosage units containing from about 10-300 mg/kg of 5-trifluoromethyldeoxycytidine, from about 20-100 mg/kg of body weight of tetrahydrouridine and from about 5-50 mg/kg of 5-benzylacyclouridine.

9. A halogenated cytidine or a deoxycytidine, a cytidine deaminase inhibitor and/or a uridine phosphorylase inhibitor for use in suitable dosage form to induce tumor regression.

10. A method of inducing regression and/or inhibiting growth of tumor cells in a mammal comprising administering to said mammal afflicted with said tumor cells effective amounts of the composition of any of Claims 1 to 6.

Claims for the following Contracting State : ES

1. The use of a halogenated cytidine or deoxycytidine, a cytidine deaminase inhibitor, and a uridine inhibitor for the manufacture of a composition for inducing tumor regression or inhibiting tumor growth.

2. The use according to claim 1 wherein pharmaceutically effective amounts of each of the three compounds are used to manufacture a unit dose composition.

3. The use according to claim 1 or claim 2 wherein the cytidine is 5-fluorodeoxycytine, 5-trifluoromethyldeoxycytidine or 5-fluorocytidine.

4. The use according to any preceding claim wherein the cytidine deaminase inhibitor is tetrahydrouridine and the uridine phospharylase inhibitor is 5-benzylacyclouridine.

5. The use according to claim 4 when dependent upon claim 3 wherein the composition contains 5-fluorodeoxycytidine, tetrahydrouridine and 5-benzylacyclouridine in such concentration ratios as to provide dosage units containing from about 8-15 mg/kg of body weight of 5-fluorodeoxycytidine; from about 10-50 mg/kg of body weight of tetrahydrouridine and from about 5-50 mg/kg of body weight of 5-benzylacyclouridine.

6. The use according to claim 4 when dependent upon claim 3 wherein the composition contains 5-trifluoromethyldeoxycytidine, tetrahydrouridine and 5-benzylacyclouridine in such concentration ratios as to provide dosage units containing from about 10-300 mg/kg of body weight of 5-trifluoromethyldeoxycytidine, from about 20-100 mg/kg of body weight of tetrahydrouridine and from about 5-50 mg/kg of body weight of 5-benzylcyclouridine.

7. The use according to any preceding claim comprising the further step of using a pharmaceutically acceptable carrier.

8. A kit comprising:

    a carrier including a series of compartments adapted to receive a series of containers in close confinement.

a first container containing 5-fluorodeoxycytidine,

a second container containing tetrahydrouridine, and

a third container containing 5-benzylacyclouridine;

said compounds being present in such concentration ratios as to provide dosage units containing from about 8-15 mg/kg of body weight of 5-fluorodeoxycytidine, from about 10-50 mg/kg of body weight of tetrahydrouridine and from about 5-50 mg/kg of body weight of 5-benzylacyclouridine.

9. A kit comprising:

a carrier including a series of compartments adapted to receive a series of containers in close confinement,

a first container containing 5-trifluoromethyldeoxycytidine,

a second container containing tetrahydrouridine,

a third container containing 5-benzylacyclouridine;

said compounds being present in such concentration ratios as to provide dosage units containing from about 10-300 mg/kg of 5-trifluoromethyldeoxycytidine, from about 20-100 mg/kg of body weight of tetrahydrouridine and from about 5-50 mg/kg of 5-benzylacyclouridine.

10. A process for preparing a pharmaceutical composition comprising the step of mixing a halogenated cytidine or deoxycytidine, a cytidine deaminase inhibitor, a uridine inhibitor, and a pharmaceutically acceptable carrier together.

FIG.I

FIG.2

European Patent Office

PARTIAL EUROPEAN SEARCH REPORT
which under Rule 45 of the European Patent Convention
shall be considered, for the purposes of subsequent
proceedings, as the European search report

Application number

EP 88 10 6153

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE-A-3 007 777 (PCR INC.)<br>* Page 2, lines 15-30; claims 5-7 *<br>-- | 1-9 | A 61 K 31/70 |
| D,A | US-A-4 613 604 (SHIH H. CHU)<br>* Column 2, lines 45-65 *<br>--------------- | 1-9 | |

TECHNICAL FIELDS
SEARCHED (Int. Cl.4)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9

Claims searched incompletely:

Claims not searched: 10

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 21-07-1988 | BRINKMANN |